# EUROPEAN PATENT SPECIFICATION

(11) **EP 4 085 824 B1**
(45) Date of publication and mention of the grant of the patent: **06.05.2026**
(21) Application number: 21759600.6
(22) Date of filing: 02.03.2021
(51) Int. Cl.: A61B 5/00, A61B 5/145, A61B 5/15, A61B 5/155

(54) **METHOD FOR TRANSMITTING OR RECEIVING BIOMETRIC INFORMATION IN CONTINUOUS BLOOD GLUCOSE MEASUREMENT SYSTEM**
VERFAHREN ZUM SENDEN ODER EMPFANGEN VON BIOMETRISCHEN INFORMATIONEN IN EINEM KONTINUIERLICHEN BLUTZUCKERMESSSYSTEM
PROCÉDÉ D'ÉMISSION OU DE RÉCEPTION D'INFORMATIONS BIOMÉTRIQUES DANS UN SYSTÈME DE MESURE CONTINUE DE LA GLYCÉMIE

(30) Priority: 28.02.2020 KR 20200025385
(43) Date of publication of application: 09.11.2022
(73) Proprietor: i-Sens, Inc., Seoul 06646 (KR)
(72) Inventor: YOU, Choong Beom, Seoul 06646 (KR); PARK, Hyo Seon, Seoul 06646 (KR)
(74) Representative: BCKIP Part mbB
(86) International application number: PCT/KR2021/002547
(87) International publication number: WO 2021/172968

(56) References cited:
- EP-A1- 3 135 195
- EP-A1- 3 984 447
- WO-A1-2017/108996
- WO-A1-2021/015389
- JP-A- 2004 234 622
- JP-A- 2012 064 019
- KR-A- 20070 031 810
- KR-A- 20180 132 555
- US-A1- 2014 321 246
- US-A1- 2015 356 490
- US-A1- 2016 335 409

## Description

### TECHNICAL FIELD

The present disclosure generally relates to a method for transmitting and receiving biometric information between a sensor transmitter and a communication terminal in a continuous blood glucose measurement system, in more detail, a method for transmitting and receiving biometric information, the method which does not determine whether biometric information has been successfully transmitted and received between a sensor transmitter and a communication terminal at every regular communication cycle and determines whether unreceived biometric information exists only during a non-receipt communication cycle relatively greater than a regular communication cycle, and, when the unreceived biometric information exists, receives the unreceived biometric information, thereby reducing the computational load required to determine whether unreceived biometric information exists at every regular communication cycle and the energy waste required to receive the unreceived biometric information at every regular communication cycle.

### BACKGROUND

Diabetes is a chronic medical condition that is common in modern people, and in the Republic of Korea, there are 2 million diabetes patients, about 5% of the total population.

Diabetes occurs when the absolute level of the sugar level in blood is high due to the absolute deficiency or relative insufficiency of insulin, produced by the pancreas, caused by various reasons such as obesity, stress, poor eating habits, and inherited hereditary factors and imbalance regarding glucose in the blood.

The blood usually contains a certain concentration of glucose, and tissue cells gain energy from the glucose.

However, when the glucose is increased excessively more than needed, the glucose cannot be properly stored in the liver, muscle, or adipose tissue and is accumulated in the blood, because of this, patients with diabetes maintain a much higher blood glucose level than normal people, and as excessive blood glucose passes through the tissues and is discharged into the urine, it results in deficiency of glucose, which is absolutely necessary for all tissues of the body, thereby causing abnormalities in respective body tissues.

Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown, and further progression of diabetes leads to complications such as visual disturbances, hypertension, kidney disease, paralysis, periodontal disease, muscle spasms and neuralgia, as well as gangrene.

In order to diagnose diabetes beforehand and manage to prevent the progression of diabetes into complications associated therewith, systematic blood glucose measurement and treatment should be performed.

Diabetes need to constantly measure blood glucose for management, so the demand for devices related to blood glucose measurement is steadily increasing. It has been confirmed through various studies that, when diabetic patients strictly control the management of blood glucose, the incidence of complications of diabetes is significantly reduced. Accordingly, it is very important for diabetic patients to measure blood glucose regularly for blood glocuse management.

In general, a finger prick type method is mainly used for blood glucose control in diabetic patients. This blood prick type method helps diabetic patients to manage their blood glucose, but because only the result at the time of measurement is displayed, there is a problem that it is difficult of precisely monitoring the blood glucose level that changes frequently. In addition, since the blood prick type blood glucose meter needs to collect blood every time to measure blood glucose frequently during the day, there is a problem in that the burden of blood collection is huge for diabetic patients.

Diabetics patients generally experience hyperglycemia and hypoglycemia, and an emergency may occur in the hypoglycemic conditions. Hypoglycemia occurs when sugar content is not kept for a long time, and the patients may become unconscious or die in a worst case. Accordingly, rapid discovery of the hypoglycemic condition is critically important for diabetics. The figure prick type blood glucose meter intermittently measuring glucose have limited ability to accurately measure blood glucose levels.

Recently, to overcome such a drawback, continuous glucose monitoring systems (CGMSs) inserted into the human body to measure a blood glucose level every few minutes have emergency situation.

The continuous glucose monitoring system includes a sensor transmitter configured to be attachable to a body part of a user and measure blood glucose by extracting body fluid, a communication terminal configured to output the received blood glucose level, and so on. The sensor transmitter measures the blood glucose of the user in a status that a sensor is inserted to a human body for a certain period, for example, fifteen (15) days, and generates blood glucose information. The sensor transmitter periodically generates blood glucose information, and the communication terminal periodically receives and outputs the blood glucose information so that the user can check the received blood glucose information.

In the continuous blood glucose measurement system described above, the sensor transmitter and the communication terminal transmit and receive blood glucose information in a wired communication type or a wireless communication type, and the communication terminal must continuously receive transmission packets from the sensor transmitter without loss.

However, it may occur that the communication terminal cannot continuously receive blood glucose information from the sensor transmitter due to temporary communication disconnection between the sensor transmitter and the communication terminal or the user's inexperienced operation, and as a result, the user may be unable to continuously monitor the user's blood glucose information through the communication terminal.

In this case, the blood glucose information that has not been received must be acquired, and only blood glucose information that the user determines to be necessary, except for unreceived blood glucose information that the user determines to be unnecessary, must be received before the blood glucose information stored in the sensor transmitter is deleted and there is demand for a method for receiving unreceived blood glucose information without wasting limited energy.

US 2016/335409 discloses a method for backfilling glucose data, wherein glucose values are associated with a consecutively numbered value.

### DETAILED DESCRIPTION OF DISCLOSURE

### Technical Problem

To solve the problem of the conventional method of transmitting and receiving biometric information described above, the purpose of the present disclosure may be for providing a 3 biometric information unreceived from a sensor transmitter by a communication terminal exists, and, when the unreceived biometric information exists, receiving the unreceived biometric information.

Another purpose of the present disclosure is for providing a method for transmitting and receiving biometric information, the method which does not determine whether biometric information has been successfully transmitted and received between a sensor transmitter and a communication terminal at every regular communication cycle transmitting the biometric information from the sensor transmitter to the communication terminal and determines whether unreceived biometric information exists only during a non-receipt communication cycle.

Still another purpose of the present disclosure is for providing a method for transmitting and receiving biometric information, the method capable of, even though unreceived biometric information exists, selectively receiving the unreceived biometric information only when a user requests.

### Solution to Problem

To accomplish the purpose of the present disclosure, according to an embodiment of the present disclosure, a method for transmitting and receiving biometric information according to claim 1 is provided.

### Advantageous Effects of Invention

A method for transmitting and receiving biometric information according to embodiments of the present disclosure has the following effects.

First, a method for transmitting and receiving biometric information does not determine whether biometric information has been successfully transmitted and received between a sensor transmitter and a communication terminal every regular communication cycle transmitting the biometric information from the sensor transmitter to the communication terminal and determines whether unreceived biometric information exists only during a non-receipt communication cycle relatively greater than a regular communication cycle, thereby reducing the process load consumed for determining whether unreceived biometric information exists at every regular communication cycle and the energy waste consumed for receiving the unreceived biometric information at every regular communication cycle.

Second, a method for transmitting and receiving biometric information according to embodiment of the present disclosure generates a biometric information message transmitted and received between a sensor transmitter and a communication terminal at every regular communication period to include a biometric information identifier together with the biometric information therein, thereby determining biometric information unreceived during a non-receipt communication cycle based on the biometric information identifier and selectively receiving only unreceived biometric information.

Third, a method for transmitting and receiving biometric information according to an embodiment of the present disclosure outputs an indicator to a user when unreceived biometric information exists during a non-receipt communication cycle, and receives the unreceived biometric information only when the user requests the unreceived biometric information based on the indicator, thereby selectively providing only unreceived biometric information determined to be necessary by the user among unreceived biometric information.

Fourth, a method for transmitting and receiving biometric information according to an embodiment of the present disclosure controls to delete an indicator when a user command for requesting unreceived biometric information is not inputted until a set time elapses after the indicator is outputted or when the user rejects the receipt of the unreceived biometric information, thereby reducing the user's inconvenience caused by unnecessary unreceived biometric information.

### BRIEF DESCRIPTION OF THE DRAWINGS

FIG. 1 is a schematic diagram for illustrating a continuous biometric information measurement system according to an embodiment of the present disclosure.
FIG. 2 is a functional block diagram for illustrating a sensor transmitter according to an embodiment of the present disclosure.
FIG. 3 is a figure for illustrating an example of generating biometric information in a sensor transmitter.
FIG. 4 is a figure for illustrating an example of generating a transmission packet in a sensor transmitter.
FIG. 5 is a functional block diagram for illustrating a communication terminal according to an embodiment of the present disclosure.
FIG. 6 is a figure for illustrating a message transmitted and received between a sensor transmitter and a communication terminal in a method for transmitting and receiving biometric information according to an embodiment of the present disclosure.
FIG. 7 is a flowchart for illustrating a method of transmitting and receiving biometric information at regular communication cycles according to an embodiment of the present disclosure.
FIG. 8 is a flowchart for explaining a method for transmitting and receiving unreceived biometric information at every non-receipt communication cycle according to an embodiment of the present disclosure.
FIG. 9 is a figure for illustrating an example of a regular communication cycle and a non-receipt communication cycle.
FIG. 10 is a figure for illustrating a method of notifying a user whether unreceived biometric information exists according to an embodiment of the present disclosure.

### DESCRIPTION OF EMBODIMENTS OF DISCLOSURE

The technical terms used in the present disclosure are only for the purpose of describing exemplary embodiments, and they are not intended to limit the present invention. Also, unless otherwise defined, all technical terms used herein should be construed as having the same meaning as commonly understood by those skilled in the art, and should not be interpreted as being excessively inclusive or excessively restrictive. In addition, when a technical term used herein is an erroneous technical term that does not accurately represent the idea of the present invention, it should be understood as replacing the term by a technical term which can be properly understood by those skilled in the art.

Further, singular expressions used in the present specification include plural expressions unless they have definitely opposite meanings. In the present application, it shall not be construed that terms, such as "including" or "comprising", various constituent elements or steps described in the specification need to be all essentially included, and it shall be construed that some constituent elements or steps among the various constituent elements or steps may be omitted, or additional constituent elements or steps may be further included.

Also, it should be noted that the accompanying drawings are merely illustrated to easily explain the spirit of the invention, and therefore, they should not be construed to limit the spirit of the invention by the accompanying drawings.

Hereinafter, with reference to the enclosed drawings, a method for receiving and transmitting biometric information according to an embodiment of the present disclosure is described in detail.

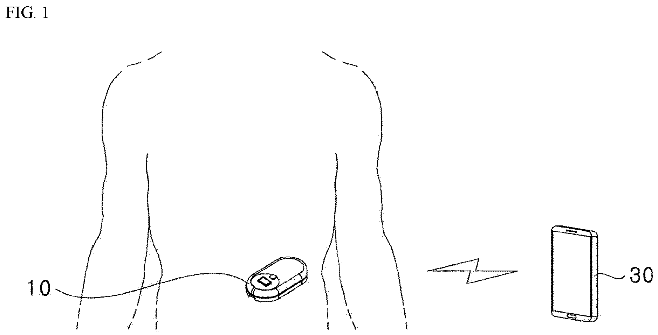

FIG. 1 is a schematic diagram for illustrating a continuous biometric information measurement system according to an embodiment of the present disclosure.

Referring to FIG. 1, the continuous biometric information measurement system (1) according to an embodiment of the present disclosure comprises a sensor transmitter (10) and a communication terminal (30).

The sensor transmitter (10) is attachable to human body and, when the sensor transmitter (10) is attached to the human body, an end portion of a sensor of the sensor transmitter (10) is inserted into skin to periodically extract body fluid of the human body and measure biometric information.

The communication terminal (30) is a terminal configured to receive biometric information from the sensor transmitter (10) and output or display the received biometric information to a user, and for example, the communication terminal (30) may be a portable terminal (such as smartphone, tablet PC, or notebook and so on) configured to communicate with the sensor transmitter (10). However, the communication terminal (30) is not limited thereto, and may be any type of a terminal to which has a communication function and program or application can be installed.

The sensor transmitter (10) transmits the biometric information in response to request of the communication terminal (30) or at predetermined times periodically, and for data communication between the sensor transmitter (10) and the communication terminal (30), the sensor transmitter (10) and the communication terminal (30) are communicationally connected to each other over a wire by an USB cable and so on or communicationally connected in an wireless communication means such as infrared communication, NFC communication, Bluetooth, etc.

Here, the communication terminal (30) determines whether biometric information that has not been received from the sensor transmitter (10) exists, and if there is unreceived biometric information, the communication terminal (30) provides an indicator for informing a user the existence of the unreceived biometric information and receives the unreceived biometric information according to the user's selection.

Depending on the field to which the present disclosure is applied, various types of biometric information such as blood glucose information may be measured and the measured biometric information can be transmitted and received between the sensor transmitter and the communication terminal.

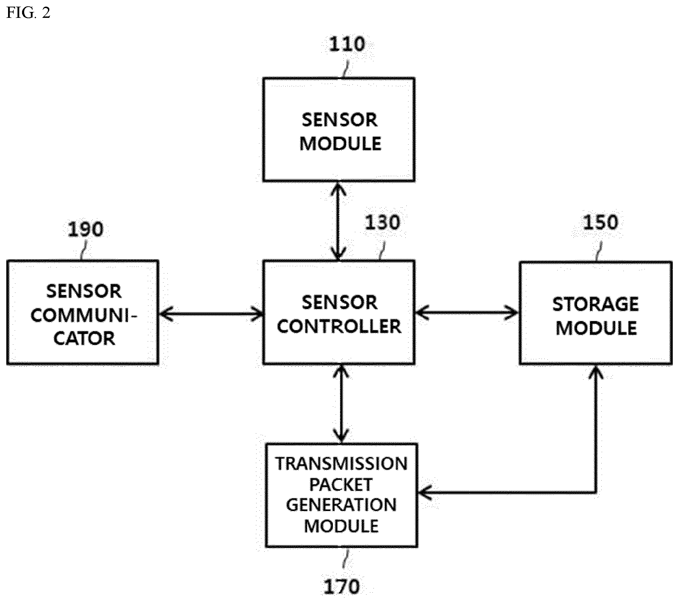

FIG. 2 is a functional block diagram for illustrating a sensor transmitter according to an embodiment of the present disclosure.

The detailed descriptions are provided as follows by referring to FIG. 2, a sensor module (110) includes a sensor, and a part of the sensor is inserted into the human body and the sensor measures biometric information.

A sensor control module or controller (130) receives the biometric information measured from the sensor module (110) and stores the received biometric information to a storage module (150). Here, the biometric information received from the sensor module (110) by the sensor controller (130) may be an analog signal, and the sensor controller (130) removes noise from the analog signal and transforms it to a digital signal to generate biometric information.

A transmission packet generation module (170) generates a transmission packet including biometric information from biometric information stored in the storage module (150), and the transmission packet generation module (170) generates a transmission packet including a biometric information identifier for identifying biometric information whenever a transmission packet is generated.

A sensor controller (130) transmits an advertisement message to the communication terminal through a sensor communicator (190) at every regular communication cycle to connect the communication with the communication terminal, and controls to transmit the generated transmission packet to the communication terminal. When a communication interval assigned to a regular communication period elapses, the sensor controller (130) immediately terminates the communication without receiving an acknowledgment message for confirming whether the transmission packet has been successfully received from the communication terminal.

On the other hand, when connecting the communication with the communication terminal and receiving a non-receipt request message from the communication terminal at every non-receipt communication cycle, the sensor controller (130) extracts unreceived biometric information corresponding to an unreceived biometric information identifier from the storage module (150) based on an unreceived biometric information identifier included in the non-receipt request message and generates an unreceived biometric information message including the extracted the unreceived biometric information. The sensor controller (150) transmits the unreceived biometric information message to the communication terminal during a communication interval assigned to the non-receipt communication cycle, and the sensor controller (150) determines whether the unreceived biometric information message is successfully transmitted based on an acknowledgement message received from the communication terminal during a communication interval.

The sensor controller (130) stores biometric information in the storage module (150) for a storage period from the time when the biometric information is generated, and, when the storage period ends, sequentially deletes the stored biometric information, but before the biometric information is deleted, biometric information generated by transmitting unreceived biometric information every non-receipt communication cycle is safely transmitted to the communication terminal.

Preferably, when unreceived biometric information is not successfully transmitted to the communication terminal through the acknowledgement message, the sensor controller (130) may delete biometric information stored in the storage module (150) except for unreceived biometric information, and then, at a next non-receipt communication cycle, the unreceived biometric information that has not been previously transmitted may be transmitted to the communication terminal.

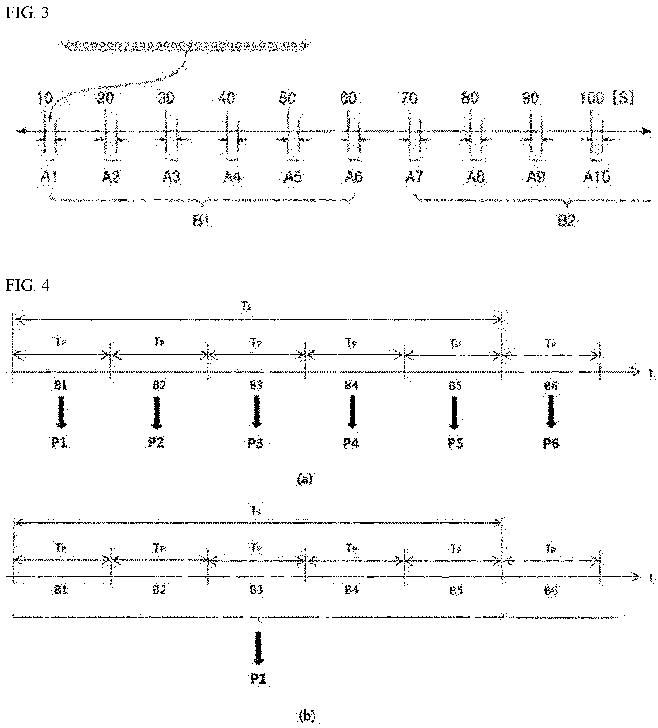

FIG. 3 is a figure for illustrating an example of generating biometric information in a sensor transmitter.

First, data regarding a biometric signal measured by a sensor transmitter are measured at every certain time period, and, for every one time period for the measurement, multiple time measurement can be performed. For example, the sensor transmitter measures biometric signal data every ten (10) seconds. At that time, every time when one unit measurement is performed, biometric signals are measured thirty (30) times, and the time consumed for measuring the biometric signals may be one (1) second. Accordingly, the sensor module measures thirty (30) analog biometric signal data every ten (10) seconds.

Accordingly, for example, blood glucose information can be measured every ten (10) seconds, such as thirty (30) times measurements of biometric information between 2 o'clock 14 minute 25 second and 2 o'clock 14 minute 26 second PM, and another thirty (30) times measurements of biometric information between 2 o'clock 14 minute 35 second and 2 o'clock 14 minute 36 second PM.

The measured biometric signal data is transformed into a digital signal. The sensor transmitter calculates one average value every ten (10) seconds by calculating an average value of thirty (30) biometric information data transformed to the digital signal by a trimmed average calculation way. At that time, seven (7) highest data and seven (7) lowest data among the thirty (30) biometric information data are removed and an average value (A) of remaining sixteen (16) data is calculated.

The trimmed average value (A) calculated by way of being described above can be generated every ten (10) seconds, and, as illustrated, six (6) trimmed average values (A1 to A6) can be generated for one (1) minute.

Additionally, six (6) trimmed average values (A1 to A6) are generated for one (1) minute, and a second trimmed average value (B1) is generated using the generated six (6) trimmed average values (A1 to A6). At that time, the generated second trimmed average value (B1) is calculated by removing a maximum value and a minimum value among six (6) trimmed average values (A1 to A6) and calculating an average of remaining four values. Accordingly, biometric information is generated from one second trimmed average value (B) for one (1) minute.

The biometric information data generated every (1) minute is stored in the sensor transmitter, and the stored biometric information can be generated as a transmission packet to be transmitted to the communication terminal at a regular communication cycle.

FIG. 4 is a figure for illustrating an example of generating a transmission packet in a sensor transmitter, and regarding an example of generating a transmission packet with reference to FIG. 4(a), biometric information (B1, B2, B3, B4, B5, B6, ...) is generated sequentially at each set biometric information generation period (T_{P}), and, each time the biometric information is generated, a transmission packet (P1, P2, P3, P4, P5, P6) having corresponding biometric information is generated. When the transmission packet is generated, a series of unique identifiers are allocated according to order of the generation of the transmission packets, and the transmission packet is generated to include the identifier of the transmission packet and biometric information. Preferably, a sequence sequentially increasing according to order of the generation of the transmission packets may be allocated as a biometric information identifier, or the time of the generation of the transmission packet may be allocated as a biometric information identifier.

The generated transmission packets (P1, P2, P3, P4, P5, P6) are stored in a storage module, and when a set regular communication cycle (T_{S}) is reached, the transmission packets (P1, P2, P3, P4, P5) stored in the storage module are respectively transmitted to the communication terminal.

Regarding another example of generating a transmission packet with reference to FIG. 4(b), biometric information (B1, B2, B3, B4, B5, B6, ...) is generated sequentially at each set biometric information generation period (T_{P}) and is stored in the storage module every time the biometric information is generated, and when a set regular communication cycle (T_{S}) is reached, a transmission packet (P1) including all biometric information stored in the storage module for the regular communication cycle is generated and the generated transmission packet (P1) is transmitted to the communication terminal.

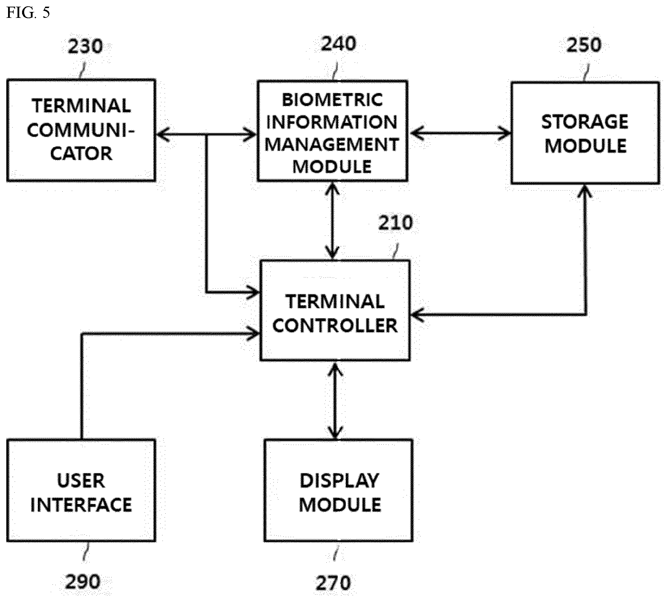

FIG. 5 is a functional block diagram for illustrating a communication terminal according to an embodiment of the present disclosure.

Referring to FIG. 5, when an advertisement message transmitted from a sensor transmitter at every set regular communication cycle is received through a terminal communicator (230), a terminal controller (210) connects the communication with the sensor transmitter through the terminal communicator (230). When being connected to the communication with the sensor transmitter, the terminal controller (210) receives biometric information from the sensor transmitter through the terminal communicator (230), and stores the received biometric information to the storage module (250). The terminal controller (210) terminates the communication with the sensor transmitter if the communication interval elapses, or terminates the communication with the sensor transmitter if the receipt of transmission packets from the sensor transmitter is completed even before the communication interval elapses.

Meanwhile, for each set non-receipt communication cycle, a biometric information management module (240) determines whether there is biometric information that has not been received during a non-receipt communication cycle based on an identifier of biometric information stored in the storage module (250). When unreceived biometric information exists, the terminal controller (210) generates a non-receipt request message including an unreceived biometric information identifier and transmits it to the sensor transmitter through the terminal communicator (230).

Here, the biometric information management module (240) sorts biometric information stored in the storage module (250) in order of their identifiers during the non-receipt communication cycle, and searches for a non-continuous biometric information identifier based on biometric information identifiers and determines biometric information with the non-continuous biometric information identifier as unreceived biometric information.

Preferably, when the unreceived biometric information exists, the terminal controller (210) generates an indicator indicating the existence of the unreceived biometric information, the number of unreceived biometric information, and so on, and displays information on the generated indicator on a display module (270) to inform the user that the unreceived biometric information exists. When a user command for receiving the unreceived biometric information is inputted from a user interface (290), a non-receipt request message including the unreceived biometric information identifier is generated and transmitted to the sensor transmitter through the terminal communicator (230) according to the user command.

When receiving unreceived biometric information from the sensor transmitter in response to the non-receipt request message at every non-receipt communication cycle, the terminal controller (210) controls to store the received unreceived biometric information to the storage module (250).

Preferably, when a user command for rejecting the reception of unreceived biometric information is input in response to the displayed indicator, the terminal controller (210) deletes the displayed indicator, and does not generate a separate non-receipt request message.

Preferably, when a user command is not input within a set time from the time when the indicator is displayed, the terminal controller (210) may delete the displayed indicator and may not generate a separate non-receipt request message.

Preferably, when a user command is not input within a set time from the time when the indicator is displayed, the terminal controller (210) outputs a message for informing that the unreceived biometric information exists on the display module, and, when a user command for rejecting the reception of unreceived biometric information is input in response to the message or when a user command is not input again within a set time from the time when the message is outputted on the display module, the terminal controller (210) may delete the displayed indicator and may not generate a separate non-receipt request message.

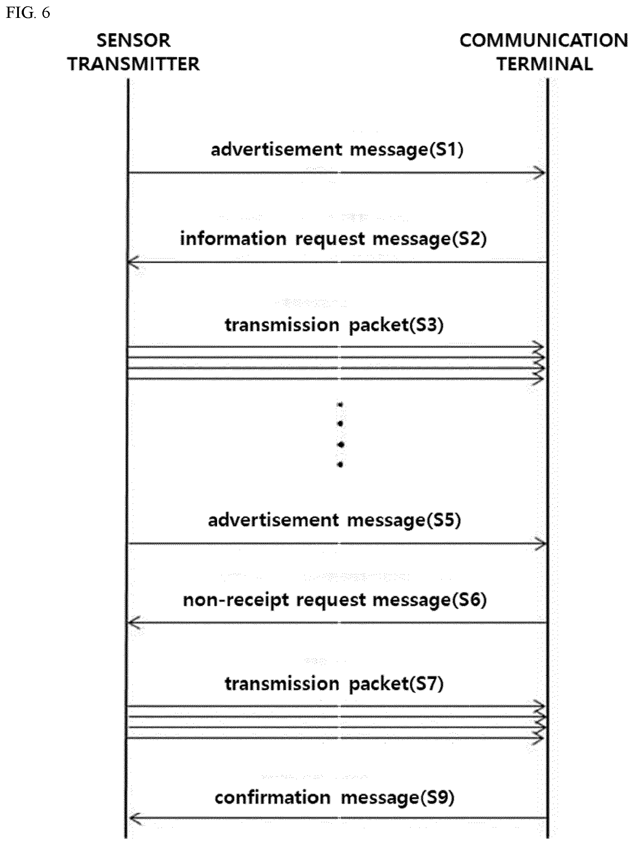

FIG. 6 is a figure for illustrating a message transmitted and received between a sensor transmitter and a communication terminal in a method for transmitting and receiving biometric information according to an embodiment of the present disclosure.

Referring to FIG. 6, the sensor transmitter transmits an advertisement message to a peripheral communication terminal at a regular communication cycle (S1).

In response to the advertisement message, the communication terminal transmits an information request message to the sensor transmitter to connect communication between the communication terminal and the sensor transmitter (S2). Preferably, the sensor transmitter adds a unique sensor transmitter identifier to the advertisement message and transmits the advertisement message to the communication terminal, and the communication terminal may authenticate the sensor transmitter based on the sensor transmitter identifier to establish a communication connection with the sensor transmitter.

When the communication is connected between the sensor transmitter and the communication terminal, the sensor transmitter transmits a transmission packet including biometric information, generated by the sensor transmitter during a set communication interval, to the communication terminal (S3). Preferably, the transmission packet includes a biometric information identifier for identifying biometric information. When the set communication interval elapses, the communication connection is terminated, or when a transmission packet is received from the communication terminal, the communication connection is terminated even before the communication interval elapses.

At every regular communication cycle, a transmission packet including biometric information, generated by the sensor transmitter by repeating steps S1, S2, S3 described above, is transmitted and received between the sensor transmitter and the communication terminal.

On the other hand, when a non-receipt communication cycle arrives, the communication terminal determines biometric information not received from the sensor transmitter during the non-receipt communication cycle, and transmits to the sensor transmitter a non-receipt request message having a non-receipt biometric information identifier for identifying unreceived biometric information (S6).

In response to the non-receipt request message, the communication between the sensor transmitter and the communication terminal is connected during a set communication interval, and the communication terminal that receives the non-receipt request message determines biometric information corresponding to the non-receipt biometric information identifier included in the non-receipt request message and transmits a transmission packet including the determined unreceived biometric information to the communication terminal during the communication interval (S7).

The communication terminal determines whether biometric information corresponding to the unreceived biometric information identifier has been received from the sensor transmitter during the communication interval and transmits a confirmation message to the sensor transmitter (S9), and, when successfully receiving biometric information corresponding to the unreceived biometric information identifier, the communication terminal generates and transmits a receipt success message to the sensor transmitter and, when the receipt of biometric information corresponding to the unreceived biometric information identifier fails, the communication terminal generates and transmits a receipt failure message to the sensor transmitter.

Preferably, the communication terminal can determine whether a non-receipt communication cycle arrives and the operation of receiving unreceived biometric information can be initiated by the communication terminal's transmitting a non-receipt request message to the sensor transmitter, but the sensor transmitter can determine whether a non-receipt communication cycle arrives and the operation of receiving unreceived biometric information can be initiated by the sensor transmitter's transmitting an advertisement message for transmitting and receiving the unreceived biometric information to the communication terminal.

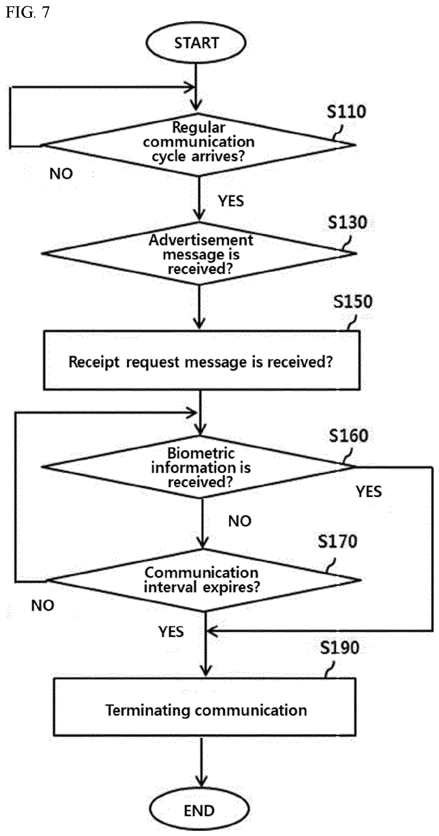

FIG. 7 is a flowchart for illustrating a method of transmitting and receiving biometric information at regular communication cycles according to an embodiment of the present disclosure.

Referring to FIG. 7, whether a regular communication cycle arrives is determined (S110).

When the regular communication cycle arrives, the communication terminal determines whether an advertisement message has been received from the sensor transmitter (S130).

When receiving the advertisement message, the communication terminal connects the communication with the sensor transmitter by transmitting a receipt request message to the sensor transmitter (S150).

When the communication between the sensor transmitter and the communication terminal is connected, the sensor transmitter transmits a transmission packet including biometric information to the communication terminal during a communication interval assigned to the regular communication cycle, and the communication terminal determines whether the transmission packet has been received from sensor transmitter during the communication interval (S160).

When receiving the transmission packet during the communication interval, the communication terminal terminates the communication connected to the sensor transmitter (S190). Preferably, when receiving the transmission packet, the communication terminal can terminate the communication connected to the sensor transmitter even before the allocated communication interval expires.

On the other hand, whether the communication interval expires is determined, and if it is before the communication interval expires, whether transmission packets are continuously received during the communication interval is determined (S170), and even if a transmission packet is not received from the sensor transmitter until the communication interval expires, the communication between the sensor transmitter and the communication terminal is terminated (S190).

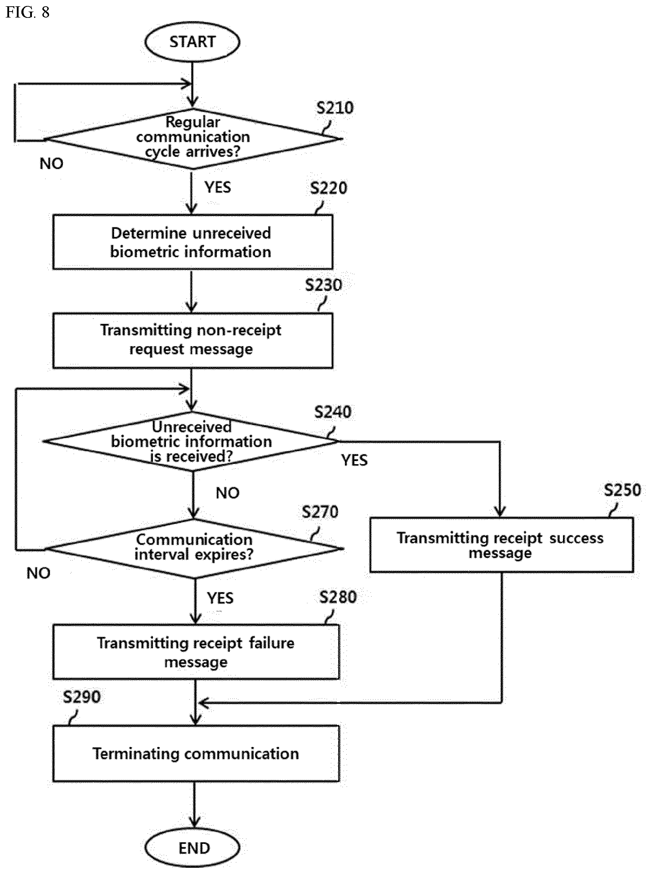

FIG. 8 is a flowchart for explaining a method for transmitting and receiving unreceived biometric information at every non-receipt communication cycle according to an embodiment of the present disclosure.

Referring to FIG. 8, it is determined whether a non-receipt communication cycle arrives (S210).

When the non-receipt communication cycle arrives, the communication terminal generates and transmits a non-receipt request message to the sensor transmitter (S220). When the non-receipt communication cycle arrives, whether unreceived biometric information exists during the non-receipt communication cycle is determined and, if there is the unreceived biometric information, an unreceived biometric information identifier is determined (S220). Each time the sensor transmitter generates biometric information, sequentially increasing biometric information identifiers are assigned, and preferably, the communication terminal determines whether unreceived biometric information exists and an unreceived biometric information identifier based on the continuity of identifiers of biometric information received during the non-receipt communication cycle. That is, when there is a missing biometric information identifier based on the continuity of identifiers of the biometric information received during the non-receipt communication cycle, it may be determined that the unreceived biometric information exists.

When unreceived biometric information exists, a non-receipt request message including an unreceived biometric information identifier is generated and the generated non-receipt request message is transmitted to the communication terminal (S230).

Communication between the sensor transmitter and the communication terminal is connected during a communication interval assigned to a non-receipt communication cycle, and whether the unreceived biometric information receives from the sensor transmitter in response to the non-receipt request message during the communication interval is determined (S240).

When all unreceived biometric information corresponding to unreceived biometric information identifiers is received, a receipt success message is generated and the generated receipt success message is transmitted to the sensor transmitter (S250), and the communication between the sensor transmitter and the communication terminal is terminated (S290). Preferably, when the communication terminal receives all unreceived biometric information, the communication terminal may terminate the communication connected to the sensor transmitter even before the allocated communication interval expires.

On the other hand, whether the communication interval has expired is determined, and if it is before the communication interval expires, whether the unreceived biometric information has been continuously received during the communication interval is determined (S270), and if all unreceived biometric information has not be received from the sensor transmitter until the communication interval expires, a receipt failure message is generated and transmitted to the sensor transmitter (S280). After transmitting the receipt failure message, the communication between the sensor transmitter and the communication terminal is terminated (S290).

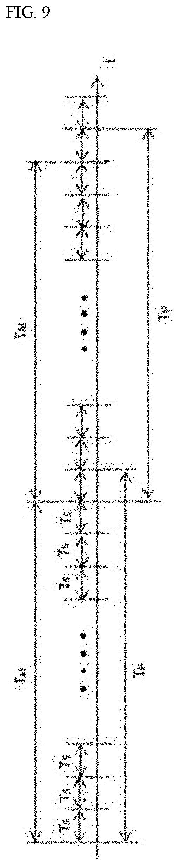

FIG. 9 is a figure for illustrating an example of a regular communication cycle and a non-receipt communication cycle.

Referring to FIG. 9, biometric information generated by the sensor transmitter is transmitted to the communication terminal at every regular communication cycle (T_{S}). The communication terminal does not send to the sensor transmitter a confirmation message indicating whether or not the biometric information transmitted at every regular communication cycle has been successfully received, and the sensor transmitter also terminates the communication with the communication terminal when a communication interval assigned to a regular communication cycle ends regardless of whether the biometric information has been successfully transmitted.

On the other hand, at every non-receipt communication cycle (T_{H}), biometric information unreceived during the non-receipt communication cycle is requested to be transmitted to the sensor transmitter, and the communication terminal generates and transmits to the sensor transmitter a confirmation message indicating whether the unreceived biometric information has been successfully received and the sensor transmitter determines whether the unreceived biometric information has been successfully transmitted based on the confirmation message.

Here, the non-receipt communication cycle (T_{H}) is set to be greater than the regular communication cycle (T_{S}), and the sensor transmitter or communication terminal does not separately check whether or not biometric information is successfully transmitted or received at each regular communication cycle or does not transmit or receive a confirmation message for checking whether or not biometric information has been successfully transmitted or received at each regular communication cycle, determines whether unreceived biometric information exists only during the non-receipt communication cycle to request and receive the unreceived biometric information, and by transmitting and receiving a confirmation message regarding whether the unreceived biometric information has been successfully received, can reduce a computational load and waste of energy for requiring to determine whether biometric information has been successfully received at every regular communication cycle.

Here, the non-receipt communication cycle (T_{H}) is set to be greater than a storage period (T_{M}) in which biometric information is stored to the sensor transmitter, and before the biometric information generated by the sensor transmitter is deleted from the sensor transmitter, the unreceived biometric information can be safely received by requesting the unreceived biometric information.

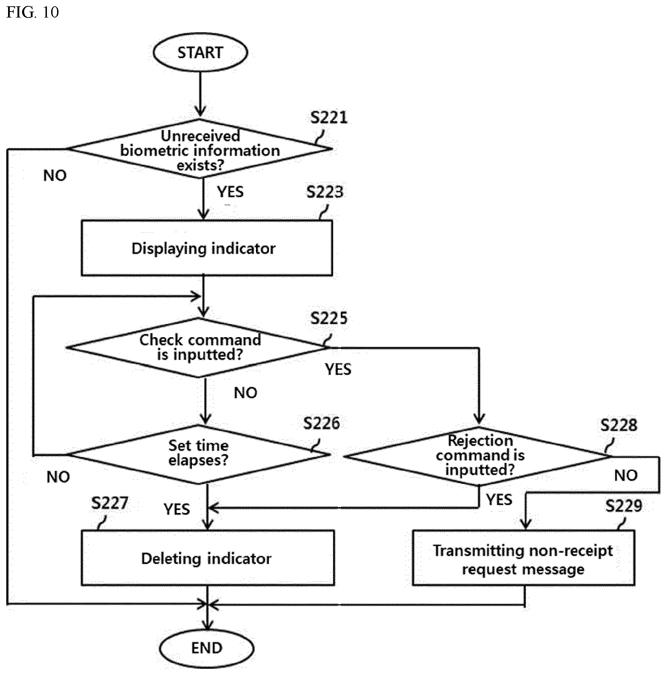

FIG. 10 is a figure for illustrating a method of notifying a user whether unreceived biometric information exists according to an embodiment of the present disclosure.

As shown in FIG. 10, when a non-receipts communication cycle arrives, whether or not unreceived biometric information exists is determined during the non-receipt communication cycle (S221).

When unreceived biometric information exists, an indicator for informing the user that the unreceived biometric information exists is output to the display module (S223). Here, the indicator may include information on whether or not unreceived biometric information exists, the number of unreceived biometric information, and whether the unreceived biometric information belongs to a dangerous area or a normal area. Here, it is characterized in that an area to which the unreceived biometric information belongs is predicted based on an area to which received biometric information adjacent to the unreceived biometric information belongs.

Whether a user command regarding whether to check unreceived biometric information is inputted through a user interface module is determined (S225), and whether the user command is not inputted until a set time elapses is determined (S226).

When the user command is not inputted until the set time elapses, the displayed indicator is deleted so as not to cause inconvenience to the user that may be caused by continuing to display the indicator (S227). Preferably, when the user command is not inputted until the set time elapses, a message informing that unreceived biometric information exists may be displayed again on the display module, and if a predetermined time elapses even after the message is displayed, the indicator may be deleted.

On the other hand, when the user command is inputted, whether the inputted user command is a user command for rejecting to check unreceived biometric information is determined (S228), and if the inputted user command is a check rejection command, the displayed indicator is deleted so as not to cause inconvenience to the user that may be caused by continuing to display the indicator (S227). However, when the inputted user command is a check command, a non-receipt request message is generated and transmitted to the sensor transmitter (S229).

In this way, an indicator for notifying the existence of unreceived biometric information is displayed to inform the user of the unreceived biometric information, but if a user command for checking the unreceived biometric information is not inputted for a set time or if the user confirms that the unreceived biometric information does not need to be checked, the indicator is deleted, and by selectively requesting unreceived biometric information only when the user requests unreceived biometric information, it is possible to reduce computational load and energy waste required for transmitting and receiving unnecessary unreceived biometric information.

Meanwhile, the exemplary embodiments of the present disclosure described above can be implemented (in embodiments not covered by the claimed methods) through programs executable at computers, and can be operated in a generalpurpose digital computer executing the programs using computer readable medium.

The above-referenced computer readable medium comprises storage medium such as magnetic storage media (e.g., ROM, floppy disks, hard disks, etc.), optical recording media (e.g., CD-ROMs, DVDs, etc.), and carrier waves (e.g., transmission through the Internet).

Although the present disclosure is described with reference to embodiments shown in the drawings in order to explain certain principles of the present disclosure by way of example, a person having ordinary skill in the art which the present disclosure relates could make various modifications and equivalent other embodiments. Accordingly, the protection scope of the present disclosure shall be defined by the claims attached hereto.

## Claims

1. A method for transmitting and receiving biometric information between a sensor transmitter (10) configured to be attachable to a body part of a user and measure the biometric information of the user and a communication terminal (30) configured to receive the biometric information from the sensor transmitter (10), the method comprising:
generating, by the sensor transmitter (10), a biometric information message including the biometric information measured through a measurement sensor and an identifier of the biometric information and transmitting the biometric information message to the communication terminal (30) at every regular communication cycle;
determining, by the communication terminal (30), whether a biometric information unreceived by the communication terminal (30) exists based on the identifier of the biometric information stored in the communication terminal (30) at every non-receipt communication cycle;
when the unreceived biometric information exists, outputting, by the communication terminal (30), an indicator for informing that the unreceived biometric information exists;
in response to the indicator, determining whether a user command for requesting the unreceived biometric information is inputted; and
when the user command is inputted, generating, by the communication terminal (30), a non-receipt request message for requesting the unreceived biometric information and transmitting the non-receipt request message to the sensor transmitter (10) at every non-receipt communication cycle,
wherein the communication terminal (30) outputs a message for informing that the unreceived biometric information exists when the user command is not input within a set time from the time when the indicator is outputted,
wherein, when the user command is not input within a predetermined time from the time when the message is outputted, the communication terminal (30) deletes the displayed indicator,
wherein the non-receipt communication cycle is greater than the regular communication cycle, and
wherein the non-receipt request message includes the one or more identifiers of the biometric information unreceived from the sensor transmitter (10) during the non-receipt communication cycle.

2. The method for transmitting and receiving the biometric information according to claim 1, wherein the communication terminal (30) determines whether unreceived biometric information exists based on continuity of identifiers of biometric information, wherein, when there is a missing biometric information identifier based on the continuity of identifiers of the biometric information, it is determined that the unreceived biometric information exists.

3. The method for transmitting and receiving the biometric information according to claim 1 or 2, wherein the sensor transmitter (10) generates and transmits the biometric information message at every regular communication cycle, and the communication terminal (30) determines whether unreceived biometric information exists at every non-receipt communication cycle which is greater than the regular communication cycle, and
wherein the non-receipt communication cycle is greater than a storage period from a time point when the biometric information is generated by the sensor transmitter (10) to a time point when the biometric information is deleted from the sensor transmitter (10).

4. The method for transmitting and receiving the biometric information according to claim 2 or 3, wherein the non-receipt request message is generated by:
when the communication terminal (30) receives the biometric information message from the sensor transmitter (10) during the non-receipt communication cycle, determining whether an identifier of the received biometric information is consecutively continued from an identifier of last biometric information stored last in the communication terminal (30) based on the identifier of the biometric information;
when the identifier of the received biometric information is not consecutively continued from the identifier of the last biometric information, determining one or more identifiers of unreceived biometric information between the received biometric information and the identifier of the last biometric information; and
when the non-receipt communication cycle ends, generating the non-receipt request message including the one or more identifiers of the unreceived biometric information.

5. The method for transmitting and receiving the biometric information according to claim 2 or 3, wherein the non-receipt request message is generated by:
sorting the biometric information received from the sensor transmitter (10) by the communication terminal (30) during the non-receipt communication cycle in order of identifiers of the received biometric information;
determining whether one or more identifiers of unreceived biometric information exists based on the identifiers of the sorted biometric information; and
when the non-receipt communication cycle ends, generating the non-receipt request message including the one or more identifiers of the unreceived biometric information.

6. The method for transmitting and receiving the biometric information according to any one of claims 1 to 5, further comprising:
determining whether the biometric information message has been received by the communication terminal (30) during a first communication interval set every regular communication cycle; and
when the biometric information message has been received by the communication terminal (30) during the first communication interval or when the first communication interval ends, terminating communication with the sensor transmitter (10),
wherein when receipt of the biometric information message for the first communication interval succeeds or when the receipt of the biometric information message for the first communication interval fails, the communication with the sensor transmitter (10) is terminated without a receipt confirmation message.

7. The method for transmitting and receiving the biometric information according to any one of claims 1 to 6, further comprising:
determining whether a non-receipt biometric information message including unreceived biometric information has been received from the sensor transmitter (10) by the communication terminal (30) during a second communication interval set every non-receipt communication cycle; and
when the non-receipt biometric information message has been received by the communication terminal (30) during the second communication interval or when the second communication interval ends, terminating communication with the sensor transmitter (10),
wherein a receipt confirmation message indicating whether the non-receipt biometric information message has been successfully received during the second communication interval is transmitted to the sensor transmitter (10).

## Patentansprüche

1. Verfahren zum Übertragen und Empfangen biometrischer Informationen zwischen einem Sensortransmitter (10), der ausgelegt ist, um an einem Körperteil eines Benutzers angebracht zu werden und die biometrischen Informationen des Benutzers zu messen, und einem Kommunikationsendgerät (30), das ausgelegt ist, um die biometrischen Informationen vom Sensortransmitter (10) zu empfangen, wobei das Verfahren umfasst:
Erzeugen einer biometrischen Informationsnachricht durch den Sensortransmitter (10), die die durch einen Messsensor gemessenen biometrischen Informationen und eine Kennung der biometrischen Informationen enthält, und Übertragen der biometrischen Informationsnachricht an das Kommunikationsendgerät (30) bei jedem regelmäßigen Kommunikationszyklus;
Bestimmen durch das Kommunikationsendgerät (30), ob biometrische Informationen, die vom Kommunikationsendgerät (30) nicht empfangen wurden, vorhanden sind, basierend auf der Kennung der im Kommunikationsendgerät (30) gespeicherten biometrischen Informationen bei jedem Kommunikationszyklus, in dem kein Empfang stattgefunden hat;
wenn die nicht empfangenen biometrischen Informationen vorhanden sind, Ausgeben eines Hinweises durch das Kommunikationsendgerät (30), zur Mitteilung, dass die nicht empfangenen biometrischen Informationen vorhanden sind;
als Reaktion auf den Hinweis, Bestimmen, ob ein Benutzerbefehl zum Anfordern der nicht empfangenen biometrischen Informationen eingegeben wurde; und
wenn der Benutzerbefehl eingegeben wurde, Erzeugen einer Nicht-Empfangs-Anforderungsnachricht durch das Kommunikationsendgerät (30) zum Anfordern der nicht empfangenen biometrischen Informationen und Übertragen der Nicht-Empfangs-Anforderungsnachricht an den Sensortransmitter (10) bei jedem Nicht-Empfangs-Kommunikationszyklus,
wobei das Kommunikationsendgerät (30) eine Mitteilung ausgibt, um darauf hinzuweisen, dass die nicht empfangenen biometrischen Informationen vorhanden sind, wenn der Benutzerbefehl nicht innerhalb einer festgelegten Zeit ab dem Zeitpunkt der Ausgabe des Hinweises eingegeben wird,
wobei, wenn der Benutzerbefehl nicht innerhalb einer vorbestimmten Zeit ab dem Zeitpunkt der Ausgabe der Mitteilung eingegeben wird, das Kommunikationsendgerät (30) den angezeigten Hinweis löscht,
wobei der Nicht-Empfangs-Kommunikationszyklus länger ist als der reguläre Kommunikationszyklus, und
wobei die Nicht-Empfangs-Anforderungsnachricht die eine oder mehrere Kennungen der biometrischen Informationen enthält, die während des Nicht-Empfangs-Kommunikationszyklus vom Sensortransmitter (10) nicht empfangen wurden.

2. Verfahren zum Übertragen und Empfangen der biometrischen Informationen gemäß Anspruch 1, wobei das Kommunikationsendgerät (30) auf der Grundlage der Kontinuität der Kennungen von biometrischen Informationen feststellt, ob nicht empfangene biometrische Informationen vorliegen, wobei, wenn auf der Grundlage der Kontinuität der Kennungen der biometrischen Informationen eine Kennung für biometrische Informationen fehlt, festgestellt wird, dass die nicht empfangenen biometrischen Informationen vorliegen.

3. Verfahren zum Übertragen und Empfangen der biometrischen Informationen gemäß Anspruch 1 oder 2, wobei der Sensortransmitter (10) die biometrische Informationsnachricht bei jedem regulären Kommunikationszyklus erzeugt und überträgt, und das Kommunikationsendgerät (30) bei jedem Nicht-Empfangs-Kommunikationszyklus, der länger als der reguläre Kommunikationszyklus ist, feststellt, ob nicht empfangene biometrische Informationen vorliegen, und
wobei der Nicht-Empfangs-Kommunikationszyklus länger ist als eine Speicherdauer von einem Zeitpunkt, an dem die biometrischen Informationen vom Sensortransmitter (10) erzeugt werden, bis zu einem Zeitpunkt, an dem die biometrischen Informationen vom Sensortransmitter (10) gelöscht werden.

4. Verfahren zum Übertragen und Empfangen der biometrischen Informationen gemäß Anspruch 2 oder 3, wobei die Nicht-Empfangs-Anforderungsnachricht erzeugt wird durch:
wenn das Kommunikationsendgerät (30) die biometrische Informationsnachricht vom Sensortransmitter (10) während des Nicht-Empfangs-Kommunikationszyklus empfängt, Bestimmen, ob eine Kennung der empfangenen biometrischen Informationen von einer Kennung der zuletzt im Kommunikationsendgerät (30) gespeicherten biometrischen Informationen fortlaufend fortgesetzt ist, basierend auf der Kennung der biometrischen Informationen;
wenn die Kennung der empfangenen biometrischen Informationen nicht von der Kennung der zuletzt gespeicherten biometrischen Informationen fortlaufend fortgesetzt ist, Bestimmen einer oder mehrerer Kennungen von nicht empfangenen biometrischen Informationen zwischen den empfangenen biometrischen Informationen und der Kennung der letzten biometrischen Informationen; und
wenn der Nicht-Empfangs-Kommunikationszyklus endet, Erzeugen der Nicht-Empfangs-Anforderungsnachricht, die die eine oder mehreren Kennungen der nicht empfangenen biometrischen Informationen enthält.

5. Verfahren zum Übertragen und Empfangen der biometrischen Informationen gemäß Anspruch 2 oder 3, wobei die Nicht-Empfangs-Anforderungsnachricht erzeugt wird durch:
Sortieren der vom Sensor-Transmitter (10) durch das Kommunikationsendgerät (30) während des Nicht-Empfangs-Kommunikationszyklus empfangenen biometrischen Informationen in Reihenfolge der Kennungen der empfangenen biometrischen Informationen;
Bestimmen, ob eine oder mehrere Kennungen von nicht empfangenen biometrischen Informationen vorhanden sind, basierend auf den Kennungen der sortierten biometrischen Informationen; und
wenn der Nicht-Empfangs-Kommunikationszyklus endet, Erzeugen der Nicht-Empfangs-Anforderungsnachricht, die die eine oder mehreren Kennungen der nicht empfangenen biometrischen Informationen enthält.

6. Verfahren zum Übertragen und Empfangen der biometrischen Informationen gemäß einem der Ansprüche 1 bis 5, ferner umfassend:
Bestimmen, ob die biometrische Informationsnachricht während eines ersten Kommunikationsintervalls, das in jedem regulären Kommunikationszyklus festgelegt ist, vom Kommunikationsendgerät (30) empfangen wurde; und
wenn die biometrische Informationsnachricht während des ersten Kommunikationsintervalls vom Kommunikationsendgerät (30) empfangen wurde oder wenn das erste Kommunikationsintervall endet, Beenden der Kommunikation mit dem Sensortransmitter (10),
wobei, wenn der Empfang der biometrischen Informationsnachricht für das erste Kommunikationsintervall erfolgreich ist oder wenn der Empfang der biometrischen Informationsnachricht für das erste Kommunikationsintervall fehlschlägt, die Kommunikation mit dem Sensortransmitter (10) ohne eine Empfangsbestätigungsnachricht beendet wird.

7. Verfahren zum Übertragen und Empfangen der biometrischen Informationen gemäß einem der Ansprüche 1 bis 6, ferner umfassend:
Bestimmen, ob eine Nicht-Empfangs-Biometrische-Informationsnachricht, die nicht empfangene biometrische Informationen enthält durch das Kommunikationsendgerät (30) vom Sensortransmitter (10), während eines zweiten Kommunikationsintervalls, das bei jedem Nicht-Empfangs-Kommunikationszyklus festgelegt ist, empfangen wurde; und
wenn die Nicht-Empfangs-Biometrische-Informationsnachricht während des zweiten Kommunikationsintervalls vom Kommunikationsendgerät (30) empfangen wurde oder wenn das zweite Kommunikationsintervall endet, Beenden der Kommunikation mit dem Sensortransmitter (10),
wobei eine Empfangsbestätigungsnachricht, die angibt, ob die Nicht-Empfangs-Biometrische-Informationsnachricht während des zweiten Kommunikationsintervalls erfolgreich empfangen wurde, an den Sensortransmitter (10) übertragen wird.

## Revendications

1. Procédé de transmission et de réception d'informations biométriques entre un émetteur de capteur (10) prévu pour pouvoir être fixé sur une partie du corps d'un utilisateur et mesurer les informations biométriques de l'utilisateur, et un terminal de communication (30) prévu pour recevoir les informations biométriques de l'émetteur de capteur (10), ledit procédé comprenant :
la génération, par l'émetteur de capteur (10), d'un message d'informations biométriques comprenant les informations biométriques mesurées par un capteur de mesure et d'un identifiant des informations biométriques, et la transmission du message d'informations biométriques au terminal de communication (30) à chaque cycle de communication régulier ;
la détermination, par le terminal de communication (30), si des informations biométriques non reçues par le terminal de communication (30) sont présentées sur la base de l'identifiant des informations biométriques stockées dans le terminal de communication (30) à chaque cycle de communication sans réception ;
si les informations biométriques non reçues sont présentées, l'émission, par le terminal de communication (30), d'un indicateur pour informer que les informations biométriques non reçues sont présentées ;
en réponse à l'indicateur, la détermination si une instruction d'utilisateur pour demander les informations biométriques non reçues est entrée ; et,
si l'instruction d'utilisateur est entrée, la génération, par le terminal de communication (30), d'un message de demande de non-réception pour demander les informations biométriques non reçues, et la transmission du message de demande de non-réception à l'émetteur de capteur (10) à chaque cycle de communication sans réception,
où le terminal de communication (30) émet un message pour informer que les informations biométriques non reçues sont présentées si l'instruction d'utilisateur n'est pas entrée dans un délai défini à partir du moment où l'indicateur est émis,
où, si l'instruction d'utilisateur n'est pas entrée dans un délai prédéterminé à partir du moment où le message est émis, le terminal de communication (30) supprime l'indicateur affiché,
où le cycle de communication sans réception est supérieur au cycle de communication régulier, et
où le message de demande de non-réception comprend le ou les identifiants des informations biométriques non reçues de l'émetteur de capteur (10) pendant le cycle de communication sans réception.

2. Procédé de transmission et de réception d'informations biométriques selon la revendication 1, où le terminal de communication (30) détermine si des informations biométriques non reçues sont présentées sur la base de la continuité d'identifiants des informations biométriques, où, si un identifiant d'informations biométriques est manquant sur la base de la continuité d'identifiants des informations biométriques, il est déterminé que les informations biométriques non reçues sont présentées.

3. Procédé de transmission et de réception d'informations biométriques selon la revendication 1 ou la revendication 2, où l'émetteur de capteur (10) génère et transmet le message d'informations biométriques à chaque cycle de communication régulier, et le terminal de communication (30) détermine si des informations biométriques non reçues sont présentées à chaque cycle de communication sans réception supérieur au cycle de communication régulier, et
où le cycle de communication sans réception est supérieur à une période de stockage entre d'un moment où les informations biométriques sont générées par l'émetteur de capteur (10) et un moment où les informations biométriques sont supprimées de l'émetteur de capteur (10).

4. Procédé de transmission et de réception d'informations biométriques selon la revendication 2 ou la revendication 3, où le message de demande de non-réception est généré par :
détermination, si le terminal de communication (30) reçoit le message d'informations biométriques provenant de l'émetteur de capteur (10) pendant le cycle de communication sans réception, si un identifiant des informations biométriques reçues est poursuivi consécutivement à partir d'un identifiant des dernières informations biométriques stockées dans le terminal de communication (30) sur la base de l'identifiant des informations biométriques ;
détermination, si l'identifiant des informations biométriques reçues n'est pas poursuivi consécutivement à partir de l'identifiant des dernières informations biométriques, d'un ou de plusieurs identifiants d'informations biométriques non reçues entre les informations biométriques reçues et l'identifiant des dernières informations biométriques ; et
par génération, si le cycle de communication sans réception se termine, du message de demande de non-réception comprenant le ou les identifiants des informations biométriques non reçues.

5. Procédé de transmission et de réception d'informations biométriques selon la revendication 2 ou la revendication 3, où le message de demande de non-réception est généré par :
tri des informations biométriques reçues de l'émetteur de capteur (10) par le terminal de communication (30) pendant le cycle de communication sans réception dans l'ordre des identifiants des informations biométriques reçues ;
détermination si un ou plusieurs identifiants d'informations biométriques non reçues sont présentés sur la base des identifiants des informations biométriques triées ; et,
lorsque le cycle de communication sans réception se termine, génération du message de demande de non-réception comprenant le ou les identifiants des informations biométriques non reçues.

6. Procédé de transmission et de réception d'informations biométriques selon l'une des revendications 1 à 5, comprenant en outre :
la détermination si le message d'informations biométriques a été reçu par le terminal de communication (30) pendant un premier intervalle de communication défini à chaque cycle de communication régulier ; et,
lorsque le message d'informations biométriques a été reçu par le terminal de communication (30) pendant le premier intervalle de communication, ou lorsque le premier intervalle de communication se termine, l'arrêt de la communication avec l'émetteur de capteur (10),
où, si la réception du message d'informations biométriques pour le premier intervalle de communication réussit ou si la réception du message d'informations biométriques pour le premier intervalle de communication échoue, la communication avec l'émetteur de capteur (10) est arrêtée sans message de confirmation de réception.

7. Procédé de transmission et de réception d'informations biométriques selon l'une des revendications 1 à 6, comprenant en outre :
la détermination si un message d'informations biométriques de non-réception comprenant des informations biométriques non reçues a été reçu de l'émetteur de capteur (10) par le terminal de communication (30) pendant un deuxième intervalle de communication défini à chaque cycle de communication sans réception ; et,
lorsque le message d'informations biométriques de non-réception a été reçu par le terminal de communication (30) pendant le deuxième intervalle de communication ou lorsque le deuxième intervalle de communication se termine, l'arrêt de la communication avec l'émetteur de capteur (10),
où un message de confirmation de réception indiquant si le message d'informations biométriques non reçu a été reçu avec succès pendant le deuxième intervalle de communication est transmis à l'émetteur de capteur (10).
